# EUROPÄISCHE PATENTSCHRIFT

(11) **EP 0 873 987 B1**
(45) Veröffentlichungstag und Bekanntmachung des Hinweises auf die Patenterteilung: **29.11.2000**
(21) Anmeldenummer: 98106830.7
(22) Anmeldetag: 15.04.1998
(51) Int. Cl.: C07C 233/25, A61K 7/13

(54) **Perfluoracylierte 3-Aminophenol-Derivate und deren Verwendung in Haarfärbemitteln**
Prefluoroacylated 3-Aminophenol derivatives and their use as hair dyeing agents
Dérivés perfluoroacylés de 3-Aminophénol et leur utilisation pour la teinture des cheveux

(30) Priorität: 24.04.1997 DE 19717293
(43) Veröffentlichungstag der Anmeldung: 28.10.1998
(73) Patentinhaber: Henkel Kommanditgesellschaft auf Aktien, 40589 Düsseldorf-Holthausen (DE)
(72) Erfinder: Rose, David, Dr., 40723 Hilden (DE); Meinigke, Bernd, Dr., 51399 Burscheid (DE); Höffkes, Horst, Dr., 40595 Düsseldorf (DE)

(56) Entgegenhaltungen:
- DE-A- 2 924 089
- DE-A- 3 638 227
- DE-A- 3 641 630
- DE-A- 19 606 644

## Beschreibung

Die Erfindung betrifft neue perfluoracylierte 3-Aminophenol-Derivate, deren Verwendung zum Färben von Keratinfasern sowie diese Verbindungen enthaltende Oxidationshaarfärbemittel.

Für das Färben von Keratinfasern, insbesondere menschlichen Haaren, spielen die sogenannten Oxidationsfärbemittel wegen ihrer intensiven Farben und guten Echtheitseigenschaften eine bevorzugte Rolle. Solche Färbemittel enthalten Oxidationsfarbstoffvorprodukte, sogenannte Entwicklerkomponenten und Kupplerkomponenten. Die Entwicklerkomponenten bilden unter dem Einfluß von Oxidationsmitteln oder von Luftsauerstoff untereinander oder unter Kupplung mit einer oder mehreren Kupplerkomponenten die eigentlichen Farbstoffe aus.

Gute Oxidationsfarbstoffvorprodukte müssen in erster Linie folgende Voraussetzungen erfüllen: Sie müssen bei der oxidativen Kupplung die gewünschten Farbnuancen in ausreichender Intensität und Echtheit ausbilden. Sie müssen ferner ein gutes Aufziehvermögen auf die Faser besitzen, wobei insbesondere bei menschlichen Haaren keine merklichen Unterschiede zwischen strapaziertem und frisch nachgewachsenem Haar bestehen dürfen (Egalisiervermögen). Sie sollen beständig sein gegen Licht, Wärme und den Einfluß chemischer Reduktionsmittel, z. B. gegen Dauerwellflüssigkeiten. Schließlich sollen sie - falls als Haarfärbemittel zur Anwendung kommend - die Kopfhaut nicht zu sehr anfärben, und vor allem sollen sie in toxikologischer und dermatologischer Hinsicht unbedenklich sein.

Als Entwicklerkomponenten werden üblicherweise primäre aromatische Amine mit einer weiteren, in para- oder ortho-Position befindlichen freien oder substituierten Hydroxy- oder Aminogruppe, Diaminopyridinderivate, heterocyclische Hydrazone, 4-Aminopyrazolonderivate sowie Aminopyrimidine und deren Derivate eingesetzt.

Als Kupplerkomponenten werden in der Regel m-Phenylendiaminderivate, Naphthole, Resorcin und Resorcinderivate, Pyrazolone und m-Aminophenole verwendet.

Allein mit einer Entwicklerkomponente oder einer speziellen Kuppler/Entwickler-Kombination gelingt es in der Regel nicht, eine auf dem Haar natürlich wirkende Farbnuance zu erhalten. In der Praxis werden daher üblicherweise Kombinationen verschiedener Entwicklerkomponenten und Kupplerkomponenten eingesetzt. Es besteht daher ständig Bedarf an neuen, verbesserten Farbstoff-Komponenten.

Es war daher die Aufgabe der vorliegenden Erfindung, neue Kuppler-Komponenten zu finden, die die an Oxidationsfarbstoffvorprodukte zu stellenden Anforderungen in besonderem Maße erfüllen.

In der deutschen Patentanmeldung DE 19 606 644.1 sind Aminophenol-Derivate und deren Verwendung als Kuppler zur Herstellung von Oxidationsfärbemitteln beschrieben. Es wurde nunmehr gefunden, daß die perfluoracylierten Derivate von 3-Aminophenolen eines bestimmten Substitutionsmusters die an Oxidations-färbemittelvorprodukte vom Kupplertyp gestellten Anforderungen in besonders hohem Maße erfüllen. So werden mit zahlreichen Entwicklern die besonders be-gehrten Färbungen im Bereich roter Nuancen erhalten. Diese Nuancen haben sich insbesondere am menschlichen Haar als sehr licht- und waschbeständig und als besonders schweißecht erwiesen.

Gegenstand der vorliegenden Erfindung sind daher perfluoracylierte 3-Aminophenol-Derivate der Formel I 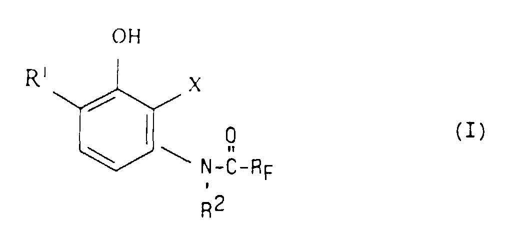 in der R¹ eine Alkylgruppe mit 1 - 4 C-Atomen, eine Hydroxyalkylgruppe mit 1 - 4 C-Atomen, eine C₁-C₄-Alkoxy-C₁-C₄-alkylgruppe oder eine Allylgruppe, R² Was-serstoff oder eine Alkylgruppe mit 1 - 4 C-Atomen oder eine Hydroxyalkylgruppe mit 2 - 4 C-Atomen, X ein Halogenatom aus der Gruppe Fluor, Chlor oder Brom und COR_{F} eine Perfluoracylgruppe mit 1 - 4 C-Atomen ist.

Die Verbindungen der Formel I sind nicht literaturbekannt. Sie lassen sich jedoch nach an sich bekannten Syntheseverfahren herstellen. Die Synthese kann z.B. von den in 6-Position mit R¹ substituierten 2-Halogen-3-aminophenolen ausgehen, die durch Acylierung mit Perfluorcarbonsäureanhydriden oder den entsprechenden Säurechloriden in die erfindungsgemäßen perfluoracylierten 3-Aminophenol-Derivate der Formel I überführt werden. Bezüglich der Einzelheiten wird auf die Herstellungsbeispiele verwiesen.

Da es sich bei den erfindungsgemäßen Verbindungen um Phenole handelt, liegen diese im alkalischen Bereich als Salze (Phenolate) vor.

Als gut und preiswert zugänglich und anwendungstechnisch besonders interessant haben sich solche Aminophenol-Derivate der Formel I erwiesen, in denen R¹ eine Methylgruppe und X Chlor ist. Schließlich erwiesen sich auch die Verbindungen der Formel I, in denen R_{F} eine Trifluormethylgruppe ist, als besonders geeignete Kuppler.

Die erfindungsgemäßen 3-Aminophenol-Derivate der Formel I eignen sich hauptsächlich zur Verwendung als Kupplerkomponente in Oxidationsfärbemitteln mit einem Gehalt an üblichen Entwicklerverbindungen zur Färbung von Keratinfasern.

Unter Keratinfasern sind dabei Pelze, Wolle, Federn und insbesondere menschliche Haare zu verstehen. Obwohl die erfindungsgemäßen Oxidationsfärbemittel in erster Linie zum Färben von Keratinfasern geeignet sind, steht prinzipiell einer Verwendung auch auf anderen Gebieten, insbesondere in der Farbphotographie, nichts entgegen.

Ein bevorzugter Gegenstand der vorliegenden Erfindung sind jedoch Oxidationsfärbemittel mit einem Gehalt an Oxidationsfarbstoffvorprodukten in einem Träger, die perfluoracetylierte 3-Aminophenol-Derivate der Formel I als Kuppler in einer Menge von 0,005 bis 10 Millimol pro 100 g des Färbemittels sowie übliche Entwicklerkomponenten und gegebenenfalls übliche Kuppler-komponenten enthalten.

Erfindungsgemäß bevorzugte Entwicklerkomponenten sind p-Phenylendiamin, p-Toluylendiamin, p-Aminophenol, o-Aminophenol, 1-(2'-Hydroxyethyl)-2,5-diaminobenzol, N,N-Bis-(2-hydroxy-ethyl)-p-phenylendiamin, 2-(2,5-Diaminophenoxy)-ethanol, 1-Phenyl-3-carboxyamido-4-amino-pyrazolon-5, 4-Amino-3-methylphenol, 2,4,5,6-Tetraaminopyrimidin, 2-Hydroxy-4,5,6-triaminopyrimidin, 4-Hydroxy-2,5,6-triaminopyrimidin, 2,4-Dihydroxy-5,6-diaminopyrimidin, 2-Dimethylamino-4,5,6-triaminopyrimidin, 2-Hydroxyethylaminomethyl-4-amino-phenol, 4,4'-Diaminodiphenylamin, 4-Amino-3-fluorphenol, 2-Aminomethyl-4-amino-phenol, 2-Hydroxymethyl-4-aminophenol, Bis-(2-hydroxy-5-aminophenyl)-methan, 1,4-Bis-(4-aminophenyl)-diazacycloheptan, 1,3-Bis(N(2-hydroxyethyl)-N(4-aminophenylamino))-2-propanol, 4-Amino-2-(2-hydroxyethoxy)-phenol sowie 4,5-Diaminopyrazol-Derivate nach EP 0 740 741 bzw. WO 94/08970 wie z.B. 4,5-Diamino-1-(2'-hydroxyethyl)-pyrazol.

Besonders bevorzugte Entwickler-komponenten sind 2,4,5,6-Tetraaminopyrimidin, 4-Hydroay-2,5,6-triaminopyrimi-din, 1-(2'-Hydroxyethyl)-2,5-diaminobenzol, p-Phenylendiamin, p-Toluylendiamin, p-Aminophenol, 3-Methyl-p-aminophenol und 2-Aminomethyl-p-aminophenol.

Erfindungsgemäß bevorzugte Kupplerkomponenten sind 1-Naphthol, Pyrogallol, 1,5-, 2,7- und 1,7-Dihydroxynaphthalin, o-Aminophenol, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2,4-diaminophenoxy)-propan, 4-Chlorresorcin, 2-Chlor-6-methyl-3-aminophenol, 2-Methylresorcin, 5-Methylresorcin, 2,5-Dimethylresorcin, 2,6-Dihydroxypyridin, 2,6-Diaminopyridin, 2-Amino-3-hydroxypyridin, 2,6-Dihydroxy-3,4-diaminopyridin, 3-Amino-2-methylamino-6-methoxypyridin, 4-Amino-2-hydroxytoluol, 2,6-Bis-(2-hydroxyethylamino)-toluol, 2,4-Diaminophenoxyethanol, 1-Methoxy-2-amino-4-(2-hydroxyethylamino)-benzol, 2-Methyl-4-chlor-5-amino-phenol, 6-Methyl-1,2,3,4-tetrahydro-chinoxalin, 3,4-Methylendioxyphenol, 3,4-Methylendioxyanilin, 2,6-Dimethyl-3-amino-phenol, 3-Amino-6-methoxy-2-methylaminophenol, 2-Hydroxy-4-aminophenoxyethanol, 2-Methyl-5-(2-hydroxyethylamino)-phenol und 2,6-Dihydroxy-3,4-dimethylpyridin.

Diese weiteren Entwickler- und Kupplerkomponenten werden üblicherweise in freier Form eingesetzt. Bei Substanzen mit Aminogruppen kann es aber bevorzugt sein, sie in Salzform, insbesondere in Form der Hydrochloride und Sulfate, einzusetzen.

Entwicklerkomponenten und Kupplerkomponenten werden im allgemeinen in etwa molaren Mengen zueinander eingesetzt. Wenn sich auch der molare Einsatz als zweckmäßig erwiesen hat, so ist ein gewisser Überschuß einzelner Oxidationsfarbstoffvorprodukte nicht nachteilig, so daß Entwicklerkomponenten und Kupplerkomponenten in einem Mol-Verhältnis von 1 : 0,5 bis 1 : 2 enthalten sein können.

Direktziehende Farbstoffe sind üblicherweise Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, Basic Yellow 57, Disperse Orange 3, HC Red 3, HC Red BN, Basic Red 76, HC Blue 2, HC Blue 12, Disperse Blue 3, Basic Blue 99, HC Violet 1, Disperse Violet 1, Disperse Violet 4, Disperse Black 9, Basic Brown 16 und Basic Brown 17 bekannten Verbindungen sowie 4-Amino-2-nitrodiphenylamin-2'-carbonsäure, 6-Nitro-1,2,3,4-tetrahydrochinoxalin, Hydroxyethyl-2-nitrotoluidin, Pikraminsäure, 2-Amino-6-chloro-4-nitrophenol 4-Ethylamino-3-nitrobenzoesäure und 2-Chloro-6-ethylamino-1-hydroxy-4-nitrobenzol. Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Weiterhin können die erfindungsgemäßen Zubereitungen auch in der Natur vorkommende Farbstoffe wie beispielsweise Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten.

Weitere in den erfindungsgemäßen Färbemitteln enthaltene Farbstoffkomponenten können auch Indole und Indoline, sowie deren physiologisch verträgliche Salze, sein. Bevorzugte Beispiele sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 6-Hydroxyindol, 6-Aminoindol und 4-Aminoindol. Weiterhin bevorzugt sind 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 6-Hydroxyindolin, 6-Aminoindolin und 4-Aminoindolin.

Es ist nicht erforderlich, daß die Oxidationsfarbstoffvorprodukte oder die fakultativ enthaltenen direktziehenden Farbstoffe jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Bezüglich der in den erfindungsgemäßen Haarfärbe- und -tönungsmitteln einsetzbaren Farbstoffe wird weiterhin ausdrücklich auf die Monographie Ch. Zviak, The Science of Hair Care, Kapitel 7 (Seiten 248-250; direktziehende Farbstoffe) sowie Kapitel 8, Seiten 264-267; Oxidationsfarbstoffvorprodukte), erschienen als Band 7 der Reihe "Dermatology" (Hrg.: Ch., Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.

Zur Herstellung der erfindungsgemäßen Färbemittel werden die Oxidationsfarbstoffvorprodukte in einen geeigneten wasserhaltigen Träger eingearbeitet. Zum Zwecke der Haarfärbung sind solche Träger z.B. Cremes, Emulsionen, Gele oder auch tensidhaltige schäumende Lösungen, z.B. Shampoos, Schaumaerosole oder andere Zubereitungen, die für die Anwendung auf dem Haar geeignet sind.

Weiterhin können die erfindungsgemäßen Färbemittel alle in solchen Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen in Form ihrer Alkali- oder Ammoniumseifen
- Ethercarbonsäuren der Formel R-O-(CH₂-CH₂O)ₓ -CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C8-C22-Carbonsäuren, wie Ölsäure, Stearinsäure, Isostearinsäure und Palmitinsäure.

Als zwitterionische Tenside werden solche oberflächenaktiven Verbindungen bezeichnet, die im Molekül mindestens eine quartäre Ammoniumgruppe und mindestens eine - COO⁽⁻⁾- oder -SO₃⁽⁻⁾-Gruppe tragen. Besonders geeignete zwitter-ionische Tenside sind die sogenannten Betaine wie die N-Alkyl-N,N-dimethyl-ammonium-glycinate, beispielsweise das Kokosalkyl-dimethylammoniumglycinat, N-Acyl-aminopropyl-N,N-dimethylammoniumglycinate, beispielsweise das Ko-kosacylaminopropyldimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der CTFA-Bezeichnung Cocamidopropyl Betaine bekannte Fettsäureamid-Derivat.

Unter ampholytischen Tensiden werden solche oberflächenaktiven Verbindungen verstanden, die außer einer C₈₋₁₈-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂₋₂₂-Fettsäuremono- und -diester von Anlagerungsprodukten von 1 bis 30 Mol Ethylenoxid an Glycerin,
- C₈₋₂₂-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungeprodukte von Ethylenoxid an Sorbitanfettsäureester
- Anlagerungsprodukte von Ethylenoxid an Fettsäurealkanolamide.

Beispiele für die in den erfindungsgemäßen Haarbehandlungsmitteln verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Coming 929 Emulsion (enthaltend ein hydroxylamino-modifiziertes Silicon, das auch als Amodimethicone bezeichnet wird), SM-2059 (Hersteller: General Electric). SLM-55067 (Hersteller: Wacker) sowie Abil®-Quat 3270 und 3272 (Hersteller: Th. Goldschmidt; diquaternäre Polydimethylsiloxane, Quaternium-80).

Alkylamidoamine, insbesondere Fettsäureamidoamine wie das unter der Bezeichnung Tego Amid®S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quaternäre Esterverbindungen, sogenannte "Esterquats", wie die unter dem Warenzeichen Stepantex® vertriebenen Dialkylammoniummethosulfate und Methyl-hydroxyalkyldialkoyloxyalkyl-ammoniummethosulfate.

Ein Beispiel für ein als kationisches Tensid einsetzbares quaternäres Zuckerderivat stellt das Handelsprodukt Glucquat®100 dar, gemäß CTFA-Nomenklatur ein "Lauryl Methyl Gluceth-10 Hydroxypropyl Dimonium Chloride".

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Weitere Wirk-, Hilfs- und Zusatzstoffe sind beispielsweise
- emulgierte Fettstoffe, z.B. emulgierte Fettalkohole mit 12 - 18 C-Atomen oder Fettsäurepartialglyceride, emulgierte Paraffine, Wachse oder emulgierte kosmetische Öle,
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylaminoethylmethacrylat-Vinylpyrrolidon-Copolymere, Vinylpyrrolidon-Imidazoliniummethochlorid-Copolymere und quaternierter Polyvinylalkohol,
- zwitterionische und amphotere Polymere wie beispielsweise Acrylamidopropyl-trimethylammoniumchlorid/Acrylat-Copolymere und Octylacrylamid/Methylmethacrylat/tert. Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copoly-mere, Methylvinylether/Maleinsäureanhydrid-Copolymere und Acrylsäure/Ethylacrylat/N-tert.Butylacrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Glucose und Maleinsäure,
- haarkonditionierende Verbindungen wie Phospholipide, beispielsweise Sojalecithin, Ei-Lecitin und Kephaline, sowie Silikonöle,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsvermittler wie Ethanol, Isopropanol, Ethylenglykol, Propylenglykol, Glycerin und Diethylenglykol,
- Farbstoffe zum Einfärben der Zubereitungen,
- Antischuppenwirkstoffe wie Piroctone Olamine und Zink Omadine,
- weitere Substanzen zur Einstellung des pH-Wertes,
- Wirkstoffe wie Panthenol, Pantothensäure, Allantoin, Pyrrolidoncarbonsäuren und deren Salze, Pflanzenextrakte und Vitamine,
- Cholesterin,
- Lichtschutzmittel,
- Konsistenzgeber wie Zuckerester, Polyolester oder Polyolalkylether,
- Fette und Wachse wie Walrat, Bienenwachs, Montanwachs, Paraffine, Fettalkohole und Fettsäureester,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex,
- Perlglanzmittel wie Ethylenglykolmono- und -distearat,
- Treibmittel wie Propan-Butan-Gemische, N₂O, Dimethylether, CO₂ und Luft sowie
- Antioxidantien.

Die Bestandteile des wasserhaltigen Trägers werden zur Herstellung der erfindungsgemäßen Färbemittel in für diesen Zweck üblichen Mengen eingesetzt; z.B. werden Emulgiermittel in Konzentrationen von 0,5 bis 30 Gew.-% und Verdickungsmittel in Konzentrationen von 0,1 bis 25 Gew.-% des gesamten Färbemittels eingesetzt.

Eine besonders bevorzugte Ausführungsform der vorliegenden Erfindung ist ein Oxidationshaarfärbemittel, das als Träger eine Cremeemulsion mit einem Gehalt von 1 - 25 Gew.-% einer emulgierten Fettkomponente oder ein Gel mit einem Gehalt von 1 - 20 Gew.-% einer Seife, jeweils bezogen auf das Haarfärbemittel, enthält.

Die oxidative Entwicklung der Färbung kann grundsätzlich mit Luftsauerstoff erfolgen. Bevorzugt wird jedoch ein chemisches Oxidationsmittel eingesetzt, besonders dann, wenn neben der Färbung ein Aufhelleffekt an menschlichem Haar gewünscht ist. Als Oxidationsmittel kommen Persulfate, Chlorite und insbesondere Wasserstoffperoxid oder dessen Anlagerungsprodukte an Harnstoff, Melamin sowie Natriumborat in Frage. Weiterhin ist es möglich, die Oxidation mit Hilfe von Enzymen zu katalysieren. Dabei können die Enzyme zur Übertragung von Luftsauerstoff auf die Entwicklerkomponente oder zur Verstärkung der Wirkung geringer Mengen vorhandener Oxidationsmittel dienen. Ein Beispiel für ein enzymatisches Verfahren stellt das Vorgehen dar, die Wirkung geringer Mengen (z.B. 1 % und weniger, bezogen auf das gesamte Mittel) Wasserstoffperoxid durch Peroxidasen zu verstärken.

Schließlich kann die oxidative Entwicklung auch durch einen Zusatz von zweiwertigen Metallen oder von lodiden katalytisch gefördert werden.

Zweckmäßigerweise wird die Zubereitung des Oxidationsmittels unmittelbar vor dem Haarefärben mit der Zubereitung aus den Oxidationsfarbstoffvorprodukten vermischt. Das dabei entstehende gebrauchsfertige Haarfärbepräparat sollte bevorzugt einen pH-Wert im Bereich von 6 bis 10 aufweisen. Besonders bevorzugt ist die Anwendung der Haarfärbemittel in einem schwach alkalischen Milieu. Die Anwendungstemperaturen können in einem Bereich zwischen 15 und 40 °C liegen. Nach einer Einwirkungszeit von ca. 30 Minuten wird das Haarfärbemittel durch Ausspülen von dem zu färbenden Haar entfernt. Das Nachwaschen mit einem Shampoo entfällt, wenn ein stark tensidhaltiger Träger, z.B. ein Färbeshampoo, verwendet wurde.

Die nachfolgenden Beispiele sollen den Erfindungsgegenstand näher erläutern.

### Beispiele

### 1. Herstellungsbeispiele für erfindungsgemäße Kuppler

### K1 : 2-Chlor-6-methyl-3-(trifluoracetylamino)-phenol

34 g 2-Chlor-6-methyl-3-aminophenol wurden in 1 l Methyl-tert.-butylether gelöst. Bei Raumtemperatur (20°C) wurden 68,1 g Trifluoressigsäureanhydrid zugetropft. Die Reaktionsmischung wurde eingeengt und das ausfallende Produkt abfiltriert. Nach Umkristallisation aus Wasser/Ethanol wurde die Verbindung in Form hellgelber Kristalle mit einem Schmelzpunkt von 110°C erhalten.

### K2 : 2-Chlor-6-methyl-3-(perfluorpropionylamino)-phenol

Die Herstellung erfolgt analog K1, jedoch unter Verwendung von Perfluor-propionsäureanhydrid. Die Substanz wurde in Form von beigefarbenen Kristallen mit einem Schmelzpunkt von 113°C erhalten.

### K3 : 2-Chlor-6-methyl-3-(perfluorbutanoylamino)-phenol

Die Herstellung erfolgt analog K1, jedoch unter Verwendung von Perfluor-buttersäureanhydrid. Die Substanz wurde in Form von beigefarbenen Kristallen mit einem Schmelzpunkt von 80°C erhalten.

### 2. Anwendungsbeispiele

Es wurden erfindungsgemäße Haarfärbemittel in Form einer Haarfärbe-Creme-emulsion der folgenden Zusammensetzung hergestellt:

| | |
|---|---|
| Cremegrundlage (1) | 50 Gew.-% |
| Entwicklerkomponente (E) | 7,5 mMol |
| Kupplerkomponente (K) | 7,5 mMol |
| Direktfarbstoff | 0 - 1 Gew.-% |
| Na ₂ SO ₃ (Inhibitor) | 1,0 Gew.-% |
| (NH ₄ ) ₂ SO ₄ | 1,0 Gew.-% |
| konz. Ammoniak-Lösung | ad pH = 10 |
| Wasser | ad 100 Gew.-% |

(1) Als Cremegrundlage wurde die folgende Zusammensetzung verwendet:

| | |
|---|---|
| Talgfettalkohol C₁₆-C₁₈ | 17,0 Gew.-% |
| Kokosfettalkohol C₁₂-C₁₈ | 4,0 Gew.-% |
| Texapon®N28 | 40,0 Gew.-% |
| Dehyton®K | 25,0 Gew.-% |
| Eumulgin B2 | 1,5 Gew.-% |
| dest. Wasser | 12,5 Gew.-% |

Darin sind folgende Handelsprodukte enthalten:
- Texapon®N28 :: Fettalkohol C₁₂-C₁₄ + 2EO-Sulfat, Na-Salz, 28 %ig, CTFA-Bezeichnung: Sodium Laureth Sulfat
- Dehyton®K :: Fettsäureamid-Betain (30 %ig) CTFA-Bezeichnung: Cocoamidopropyl-Betaine
- Eumulgin®B2 :: Cetyl-/Stearylalkohol + 20 Mol EO CTFA-Bezeichnung: Ceteareth 20

Als Entwicklerkomponenten (E) wurden die folgenden Verbindungen eingesetzt:
- E1 :: Aminophenol
- E2 :: 4-Hydroxy-2,5,6-triamino-pyrimidin
- E3 :: 2-(2-Hydroxyethyl)-1,4-phenylendiamin
- E4 :: 2-(2,5-Diamino)-phenoxyethanol
- E5 :: 2-Aminomethyl-4-aminophenol
- E6 :: 2,4,5,6-Tetraaminopyrimidin
- E7 :: 3-Methyl-4-aminophenol
- E8 :: 1,3-N,N'-Bis-(2-hydroxyethyl)-N,N'-bis-(4-aminophenyl)-diaminopropan-2-ol

Als Kupplerkomponenten wurden die Verbindungen K1, K2 und K3 (Herstell-beispiele) eingesetzt und als bekannter Kuppler wurde mitverwendet:
- K4: : 2,4-Diaminophenoxyethanol.

Als direktziehende Farbstoffe wurden mitverwendet:
- D1 :: HC-Blue No.2
- D2 :: HC-Red No. 3.

Die Bestandteile der Haarfärbe-Cremeemulsion wurden der Reihe nach miteinander vermischt. Nach Zugabe der Oxidationsfärbemittelvorprodukte und des Inhibitors sowie der Direktfarbstoffe wurde zunächst mit konzentrierter Ammoniaklösung der pH-Wert der Emulsion auf 10 eingestellt, dann wurde mit Wasser auf 100 g aufgefüllt.

Die oxidative Entwicklung der Färbung wurde mit 3 %iger Wasserstoffperoxidlösung als Oxidationslösung durchgeführt. Hierzu wurden 100 g der Emulsion mit 50g Wasserstoffperoxidlösung (3 %ig) versetzt und vermischt.

Die Färbecreme wurde auf ca. 5 cm lange Strähnen standardisierten, zu 90 % ergrauten, aber nicht besonders vorbehandelten Menschenhaars aufgetragen und dort 30 Minuten bei 32 °C belassen. Nach Beendigung des Färbeprozesses wurde das Haar gespült, mit einem üblichen Haarwaschmittel gewaschen und anschließend getrocknet.

Die Ergbnisse der Haarfärbeversuche sind in der folgenden Tabelle dargestellt:

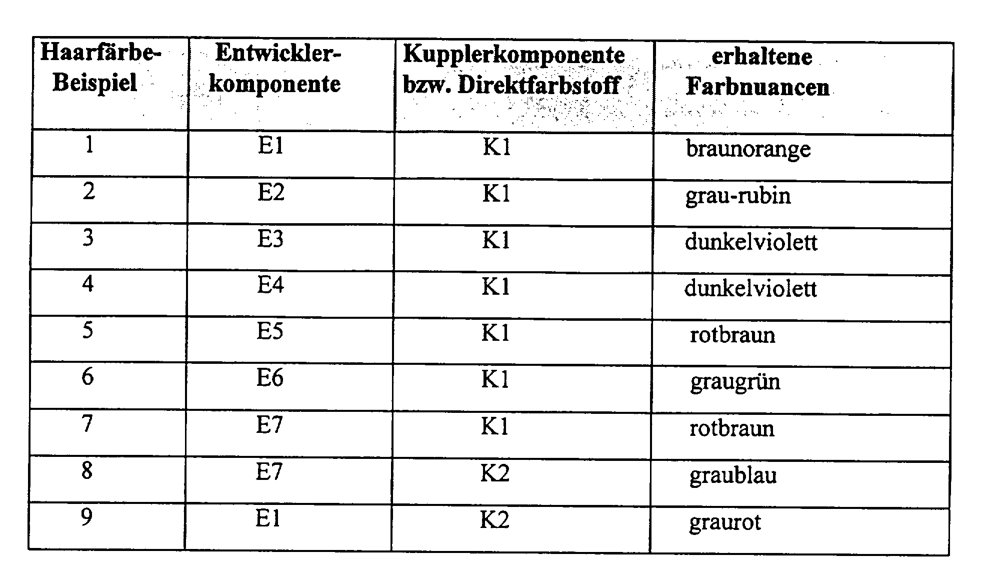

**Fortsetzung Tabelle :**

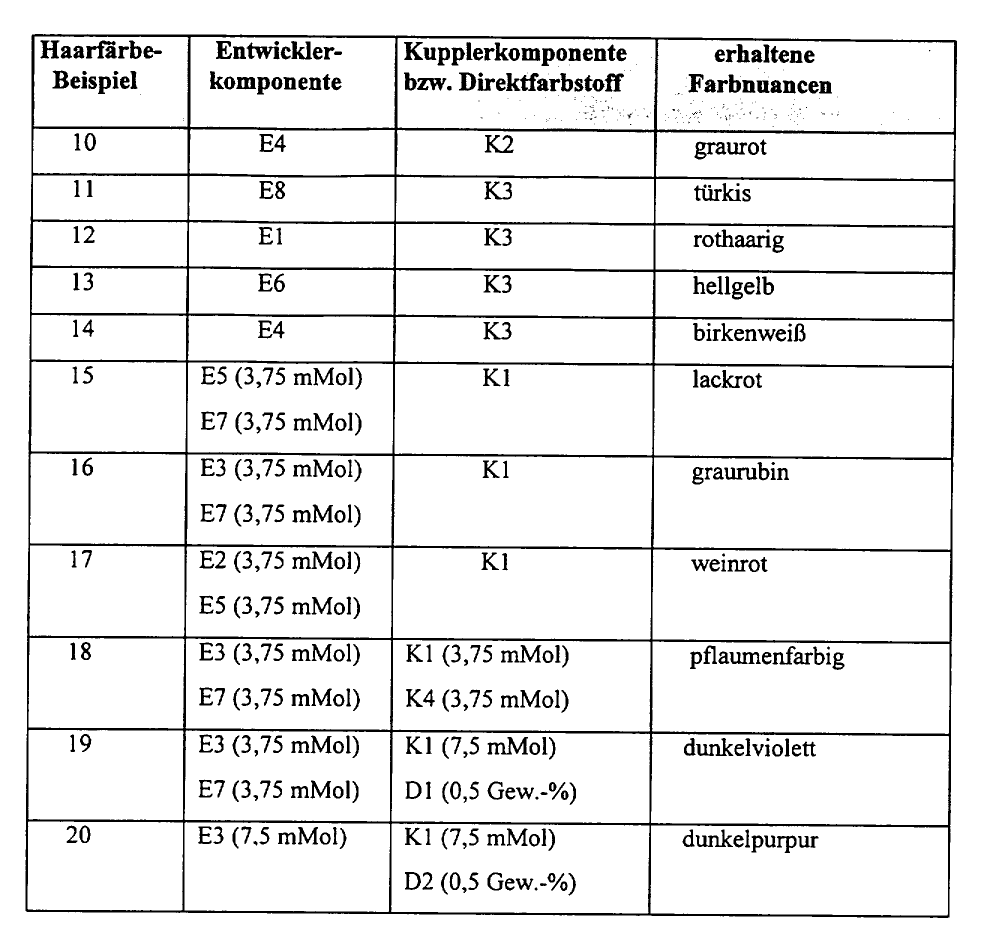

## Patentansprüche

1. Perfluoracylierte 3-Aminophenol-Derivate der Formel I 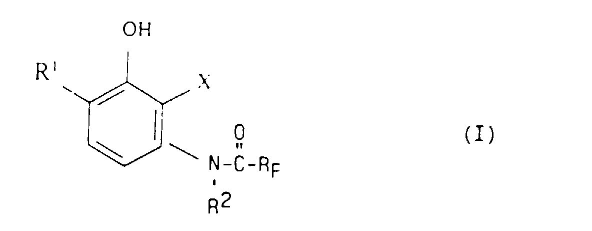 in der R¹ eine Alkylgruppe mit 1 - 4 C-Atomen, eine Hydroxyalkylgruppe mit 1 - 4 C-Atomen, eine C₁-C₄-Alkoxy-C₁-C₄-alkylgruppe oder eine Allylgruppe, R₂ Wasserstoff oder eine Alkylgruppe mit 1 - 4 C-Atomen oder eine Hydroxy- alkylgruppe mit 2 - 4 C-Atomen, X ein Halogenatom aus der Gruppe Fluor, Chlor oder Brom und COR_{F} eine Perfluoracylgruppe mit 1 - 4 C-Atomen ist.

2. 3-Aminophenol-Derivat nach Anspruch 1, dadurch gekennzeichnet, daß R¹ eine Methylgruppe und X Chlor ist.

3. 3-Aminophenol-Derivat nach Anspruch 1 oder 2, dadurch gekennzeichnet, daß R_{F} eine Trifluormethylgruppe ist.

4. Verwendung von perfluoracylierten 3-Aminophenol-Derivaten nach einem der Ansprüche 1 bis 3 als Kupplerkomponente in Oxidationsfärbemitteln mit einem Gehalt an üblichen Entwicklerverbindungen zur Färbung von Keratinfa- sern.

5. Oxidationsfärbemittel enthaltend Oxidationsfarbstoffvorprodukte in einem Träger, dadurch gekennzeichnet, daß perfluoracylierte 3-Aminophenol-Derivate der Formel I gemäß Anspruch 1 als Kuppler in einer Menge von 0,005 bis 10 Millimol pro 100 g des Färbemittels sowie übliche Entwicklerkomponenten und gegebenenfalls übliche Kupplerkomponenten enthalten sind.

6. Oxidationshaarfärbemittel nach Anspruch 5, dadurch gekennzeichnet, daß als Träger eine Cremeemulsion mit einem Gehalt von 1 - 25 Gew.-% einer emul- gierten Fettkomponente oder ein Gel mit einem Gehalt von 1 - 20 Gew.-% einer Seife, jeweils bezogen auf das gesamte Haarfärbemittel, enthalten ist.

7. Oxidationshaarfärbemittel nach einem der Ansprüche 5 oder 6, dadurch ge-kennzeichnet, daß weiterhin wenigstens ein direktziehender Farbstoff enthalten ist.

## Claims

1. Perfluoroacylated 3-aminophenol derivatives corresponding to formula I: 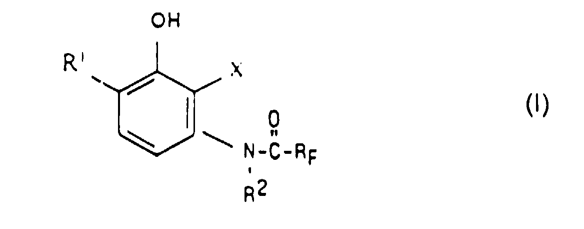 in which R¹ is a C₁₋₄ alkyl group, a C₁₋₄ hydroxyalkyl group, a C₁₋₄-alkoxy-C₁₋₄-alkyl group or an allyl group, R² is hydrogen or a C₁₋₄ alkyl group or a C₂₋₄ hydroxyalkyl group, X is a halogen atom from the group consisting of fluorine, chlorine or bromine and COR_{F} is a C₁₋₄ perfluoroacyl group.

2. A 3-aminophenol derivative as claimed in claim 1, characterized in that R¹ is a methyl group and X is chlorine.

3. A 3-aminophenol derivative as claimed in claim 1 or 2, characterized in that R_{F} is a trifluoromethyl group.

4. The use of the perfluoroacylated 3-aminophenol derivatives claimed in any of claims 1 to 3 as a secondary intermediate in oxidation colourants containing typical primary intermediates for colouring keratin fibres.

5. Oxidation colourants containing oxidation dye precursors in a carrier, characterized in that the perfluoroacylated 3-aminophenol derivatives corresponding to formula I in claim 1 are present as secondary intermediates in a quantity of 0.005 to 10 millimoles per 100 g of the colorant together with typical primary intermediates and optionally typical secondary intermediates.

6. Oxidation colourants as claimed in claim 5, characterized in that a cream emulsion containing 1 to 25% by weight of an emulsified fatty component or a gel containing 1 to 20% by weight of a soap, based on the hair colourant as a whole, is present as the carrier.

7. Oxidation hair colourants as claimed in claim 5 or 6, characterized in that at least one substantive dye is additionally present.

## Revendications

1. Dérivés perfluoroacylés de 3-aminophénol de formule I, 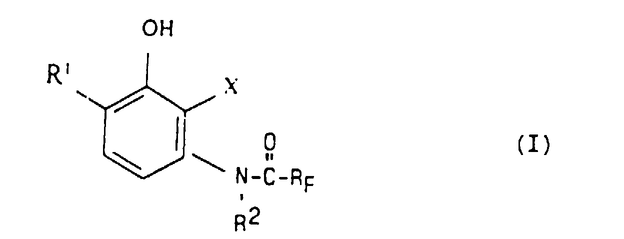 dans laquelle R¹ est un groupe alkyle ayant de 1 à 4 atomes de carbone, un groupe hydroxyalkyle ayant de 1 à 4 atomes de carbone, un groupe (alkoxy en C₁-C₄)-(alkyle en C₁-C₄) ou un groupe allyle, R₂ est de l'hydrogène ou un groupe alkyle ayant de 1 à 4 atomes de carbone ou un groupe hydroxyalkyle ayant de 2 à 4 atomes de carbone, X est un atome d'halogène choisi dans le groupe du fluor, du chlore ou du brome et, COR_{F} est un groupe perfluoroacyle ayant de 1 à 4 atomes de carbone.

2. Dérivé de 3-aminophénol selon la revendication 1,
caractérisé en ce que
R¹ est un groupe méthyle et X est un chlore.

3. Dérivé de 3-aminophénol selon la revendication 1 ou la revendication 2,
caractérisé en ce que
RF est un groupe trifluorométhyle.

4. Utilisation de dérivés de 3-aminophénol perfluoroacylés selon l'une quelconque des revendications 1 à 3
comme composant d'agent de couplage dans des colorants par oxydation, ayant une teneur en composés de développeur usuels, en vue de la coloration des fibres de kératine.

5. Colorant par oxydation contenant des produits précurseurs de colorants par oxydation dans un support,
caractérisé en ce qu'
il renferme des dérivés de 3-aminophénol perfluoroacylés de formule I conformément à la revendication 1, comme agent de couplage en une quantité allant de 0,005 à 10 millimol pour 100 g de colorant, ainsi que des composants de développeur usuels, et le cas échéant des composants usuels d'agent de couplage.

6. Colorant par oxydation pour les cheveux selon la revendication 5,
caractérisé en ce qu'il contient
comme support, une émulsion en crème ayant une teneur de 1 à 25 % en poids d'un composant gras mis en émulsion ou un gel ayant une concentration de 1 à 20 % en poids d'un savon, à chaque fois rapporté à la totalité du colorant pour cheveux.

7. Colorant par oxydation pour les cheveux, selon l'une des revendications 5 ou 6,
caractérisé en ce qu'il contient
en outre au moins un colorant direct.
